(19) Europäisches Patentamt
European Patent Office
Office européen des brevets

(11) EP 1 314 981 A2

(12) # EUROPÄISCHE PATENTANMELDUNG

(43) Veröffentlichungstag:
**28.05.2003 Patentblatt 2003/22**

(51) Int Cl.⁷: **G01N 33/533**, G01N 33/58, C07D 209/90, C07D 417/06

(21) Anmeldenummer: **02026263.0**

(22) Anmeldetag: **26.11.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **26.11.2001 DE 10157615**

(71) Anmelder: **Chromeon GmbH**
**93053 Regensburg (DE)**

(72) Erfinder:
- **Romanov, Nikolai N.**
**03087 Kiew (UA)**
- **Wolfbeis, Otto S.**
**93051 Regensburg (DE)**

(74) Vertreter: **Dey, Michael, Dipl.-Chem. Dr. et al**
**Postfach 860 820**
**81635 München (DE)**

(54) **Benzo[c,d]indol-Fluorochrome für die Markierung von Biomolekülen und Polymerpartikeln**

(57) Es werden neue reaktive Farbstoffe beschrieben, mit deren Hilfe bioorganische Moleküle wie z.B. Aminosäuren, Proteine, Antikörper, Nucleotide, aber auch Polymerpartikel fluoreszent markiert werden können. Das Verfahren beruht auf der Umsetzung eines Biomoleküls oder Partikels mit einem reaktiven Benzo [c,d]indolderivat- Chromophor. Die neuen Farbstoffe bzw. deren Konjugate können in ihrer Farbe über einen weiten Bereich eingestellt werden. Im Gegensatz zu bekannten symmetrischen Cyaninfarbstoffen enthalten sie nur eine reaktive Gruppe, sodass die Markierung ohne die bei Bireaktiven Farbstoffen störende Quervernetzung abläuft. Ihre Fluoreszenz-Quantenausbeuten sind (besonders in konjugierter Form) sehr hoch. Die Konjugate an Proteine, Oligonucleotide oder an Partikel finden Verwendung in fluoreszenz-analytischen Bestimmungsverfahren, z.B. in Immunoassays, in Hybridisierungsassays, in der Cytometrie oder im pharmazeutischen Screening.

EP 1 314 981 A2

Printed by Jouve, 75001 PARIS (FR)

## Beschreibung

Die Erfindung betrifft neue reaktive Farbstoffe sowie deren Verwendung zur Fluoreszenzmarkierung von bioorganischen Molekülen oder organischen Partikeln.

Die Fluoreszenzmarkierung von Biomolekülen spielt eine wichtige Rolle in der Bioanalytik und biologischen Forschung. Man unterscheidet zwischen Fluorophoren, die nicht-kovalent an Biomoleküle wie z. B. Proteine oder DNA binden (z. B. Interkalatoren), und solchen, die kovalent (chemisch) an Biomoleküle gebunden werden können.

Eine Vielzahl von fluoreszenten Markern wurde beschrieben. Generell handelt es sich dabei um fluoreszente Chromophore, die eine sehr reaktive Gruppe tragen, die mit einer anderen funktionellen Gruppe eines Biomoleküls eine chemische (kovalente) Bindung eingehen. Das allgemeine Reaktionsschema einer kovalenten Markierung kann wie folgt dargestellt werden: An einem Fluorophor ***F*** befindet sich eine reaktive Gruppe ***X,*** die mit einer an einem Biomolekül oder Partikel befindlichen zweiten reaktiven Gruppe ***HY*** eine chemische Reaktion eingeht. Meist wird dabei eine Gruppe vom Typ HX abgespalten.

*F* - X + HY-Biomolekül = = > *F* - Y-Biomolekül + HX

Alternativ kann man einen Farbstoff mit einer Biotingruppe versehen. Biotin bindet mit hoher Affinität ($K_d$ ca. $10^{13}$ M) an die Proteine Avidin oder Streptavidin (SA). Wenn sich das SA an einem Biomolekül befindet, kann man über die Biotin-(Strept)avidin-Bindung eine nicht-kovalente Anknüpfung von Farbstoffen an Biomoleküle erzielen.

Auf diese Weise gelingt es, einen Fluorophor ***F*** in ein Biomolekül einzuführen und dieses somit allen fluoreszenz-analytischen Verfahren zugänglich zu machen. Typische Beispiele für die Gruppen X und Y sind in Tabelle 1 angegeben.

Tabelle 1.

*Reaktivgruppen (X) an synthetischen Fluorophoren, die deren Ankopplung an spezifische Gruppen (Y) an Biomolekülen oder an Polymeren ermöglichen. Daneben haben auch die in den beiden letzten Spalten angeführten Affinitätsbindungen zwischen Biotin und Strept(avidin) große Bedeutung.*

| X (am Fluorophor) | Y (am Biomolekül oder Partikel) | Reaktionstyp (Abgangsgruppe bzw. neue Gruppe) |
|---|---|---|
| $-SO_2Cl$ | $-NH_2$ | Substitution (Abspaltung von HCl) |
| $-CO-CH_2-I$ | -SH | Substitution (Abspaltung von HI) |
| $-CO-CH_2-Br$ | -COOH | Substitution (Abspaltung von HBr) |
| -CO-O-SUI[a)] | $-NH_2$ (Protein) | Substitution (Abspaltung von NHS) |
| -N = C = S | $-NH_2$ (Protein) | Addition (zu -NH-CS-NH-) |
| -N=C=O | -OH (Alkohol) | Addition (zu -NH-CO-O-) |
| Phosphoramidit | -OH (Desoxyribose) | kovalente Bindung (P-O) |
| -Maleinimid | -SH (Protein) | kovalente Addition |
| -(Strept)avidin | -Biotin | Affinitätsbindung |
| -Biotin | (Strept)avidin | Affinitätsbindung |

a) SUI steht für die N-Succinimidoyl-Gruppe

Zu den typischen Farbstoffgruppen gehören die von Waggoner und Mitarbeitern in den US-PS 6.048.982, 5.627.027 , 5.569.766, 5.569.587, 5.486.616, 5.268.486 beschriebenen Reaktivfarbstoffe aus der Gruppe der Cyanine der folgenden allgemeinen Struktur:

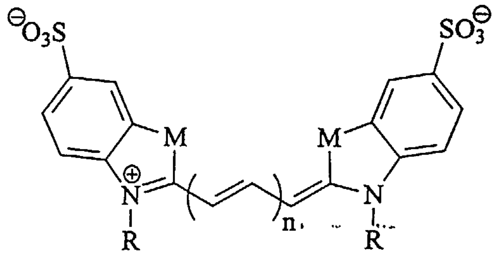

**A**

worin *M* für O, S, NR oder $C(CH_3)_2$ steht. An einem der Reste *R* befindet sich üblicherweise eine reaktive Gruppe (siehe Tabelle 1), die eine Bindung an ein Biomolekül ermöglicht.

Die Farbe derartiger Verbindungen wird primär durch *n* (typischerweise 0-3) bestimmt, weniger stark auch durch den heterocyclischen Ring oder dessen Substituenten. In den folgenden 3 Verbindungen **(B, C, D)** steigt das Absorptionsmaximum pro *n* um typischerweise 100 nm, also von 440 über 560 zu 650 nm:

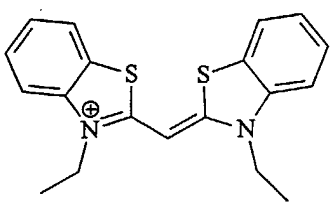

**B**

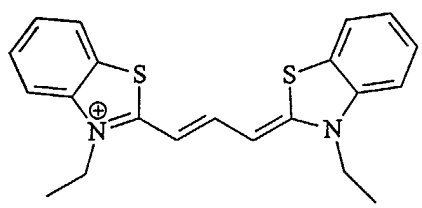

**C**

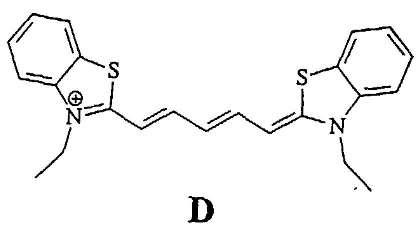

**D**

Allerdings sind Verbindungen dieser Art relativ photolabil und oxidationsempfindlich. Man hat daher nach Wegen gesucht, die Stabilität zu verbessern, indem zum Beispiel rigidisierende Ringe eingebaut wurden. Daraus resultierten z. B. folgende Verbindungen **(E, F, G):**

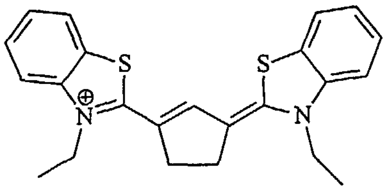

**E**

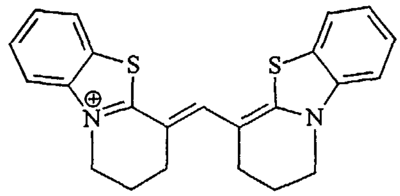

**F**

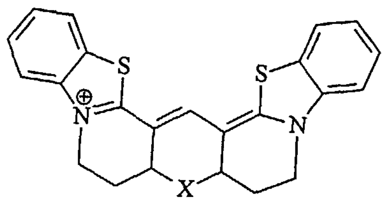

**G**

Diese sind aber sehr aufwendig herzustellen und leiden wegen ihrer Molekülgröße unter zunehmender Wasserunlöslichkeit, was sie für Anwendungen in der Bioanalytik wenig geeignet macht. Besonders nachteilig ist aber der Umstand, dass derartige Farbstoffe nur dann leicht herstellbar sind, wenn sie eine symmetrische Struktur haben. Das bedeutet für den Fall von reaktiven Markern, dass immer auch zwei reaktive Gruppen vorhanden sind. Dies ist unnötig, oft sogar unerwünscht, da es wegen der beiden Gruppen zu einer sehr nachteiligen Quervernetzung von Biomolekülen durch derartige bireaktive Farbstoffe kommen kann.

Eine Aufgabe der vorliegenden Erfindung war es deshalb neue Markerfarbstoffe bereitzustellen, welche eine hohe Photostabilität aufweisen und gleichzeitig einfach herstellbar sind. Weiterhin sollte es möglich sein, die neuen Markerfarbstoffe mit nur einer Reaktivgruppe auszustatten.

Es wurde nun gefunden, dass man unsymmetrische und monoreaktive Farbstoffe mit deutlich verbesserter Photostabiltät vergleichsweise einfach erhalten kann, wenn der Chromophor in neuartiger Weise über einen aromatischen (oder hetero-aromatischen) Ring angeordnet wird. Die neuen Farbstoffe besitzen den Vorteil, dass sie sehr photostabil sind, vorzugsweise nur eine Reaktivgruppe besitzen (entsprechend X in Tabelle 1), und dass die monoreaktiven Farbstoffe vergleichsweise leicht herstellbar sind. Sie besitzen die allgemeine chemische Struktur 1,

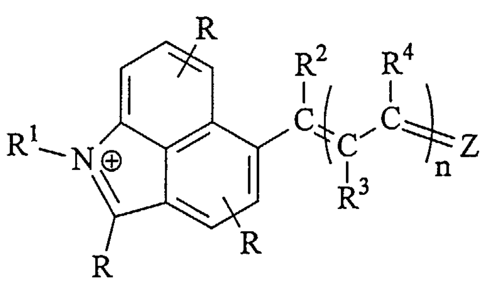

**1**

worin

R jeweils unabhängig voneinander für H oder einen beliebigen organischen Substituenten oder Ring steht, der insbesondere die Fluoreszenz des Systems nicht löscht,

$R^1$ bis $R^4$ jeweils unabhängig voneinander für H, einen gegebenenfalls substituierten, linearen, verzweigten oder cyclischen, gesättigten, oder einen oder mehrfach ungesättigten Alkylrest, oder einen gegebenenfalls substituierten Aryl-

rest stehen kann,
$R^2$ bis $R^4$ auch für eine Halogen-, Cyano- oder gegebenenfalls substituierte Aminogruppe stehen kann,
R und $R^1$ bis $R^4$ über gegebenenfalls auch heterocyclische Ringe miteinander verbunden sein können;
*Z* für einen beliebigen zweiwertigen aromatischen oder heteroaromatischen Rest steht, der auch einen Rest *R*, wie er oben definiert ist, enthalten kann,
*n* Werte zwischen 0 und 3 annehmen kann, und
zumindest ein Rest R, bzw. $R^1$ bis $R^4$ eine beliebige Gruppe (einschließlich einer Biotingruppe) enthält, die eine Markierung eines Biomoleküls oder eines Partikels ermöglicht.

R steht bevorzugt für einen Substituenten mit 1 bis 30 C-Atomen, vorzugsweise 1 bis 12 C-Atomen oder für ein Ring(system) mit 5 bis 30, insbesondere 6 bis 12 C-Atomen. Besonders geeignete Reste sind beispielsweise $C_1$-$C_{12}$-Alkylreste sowie Arylreste, insbesondere der Phenylrest. Vorzugsweise werden die Reste R so ausgewählt, dass sie die Fluoreszenz des Systems nicht löschen.

$R^1$ bis $R^4$ stehen bevorzugt für einen $C_1$-$C_{12}$-Rest und am meisten bevorzugt für H. Die Substituenten am Alkylrest und Arylrest werden vorteilhafterweise ausgewählt aus Hydroxy, Halogen, $C_1$-$C_6$-Alkoxy sowie Amino.

$R^2$ bis $R^4$ können weiterhin auch für Halogen, Cyano oder eine gegebenenfalls substituierte Aminogruppe stehen, wobei die Substituenten hier vorteilhafterweise ausgewählt werden aus $C_1$-$C_8$-Kohlenwasserstoffgruppen, insbesondere Alkylgruppen und $C_1$-$C_8$-Hydroxyalkylgruppen.

Zwei oder mehr der Reste R, $R^1$, $R^2$, $R^3$ und $R^4$ können auch miteinander verbunden sein, um Ringsysteme zu bilden, insbesondere heterocyclische Ringe, welche mindestens ein Atom, ausgewählt aus N, O, S oder P enthalten.

Z in Formel 1 steht für einen zweiwertigen aromatischen oder heteroaromatischen Rest, welcher vorzugsweise 5 bis 30 C-Atome, insbesondere 5 bis 12 C-Atome aufweisen kann. Die Heteroatome sind vorteilhafterweise ausgewählt aus N, O, S und P. Der Rest Z kann wiederum ein- oder mehrfach substituiert sein mit Resten R, wie sie oben definiert sind.

Die erfindungsgemäßen Farbstoffe zeichnen sich weiterhin dadurch aus, dass mindestens ein Rest R, $R^1$, $R^2$, $R^3$ oder $R^4$ eine Gruppierung enthält, die eine Markierung eines Biomoleküls oder eines Partikels ermöglicht. Eine solche Gruppierung ist insbesondere geeignet, eine kovalente Bindung mit einer reaktiven Gruppe an einem Biomolekül oder einem Partikel einzugehen oder ist in der Lage mit einem Biomolekül oder einem Partikel Wechselwirkungen einzugehen. Geeignete Reaktivgruppen, die eine Markierung eröglichen, sind beispielsweise $-SO_2Cl$, $-CO-CH_2-I$, $-CO-CH_2-Br$, -CO-O-N-Succinimidoyl, -N = C = S, -N = C = O, Phosphoramidit, -Maleinimid, (Strept)avidin oder Biotin.

Überraschenderweise wird durch den neuen Chromophor auch ein weiterer positiver Effekt erzielt, nämlich eine Verlängerung des Absorptionsmaximums in den rotwelligen Bereich. Dies zeigt ein Vergleich der Absorptionsmaxima der beiden folgenden Farbstoffe **H** bzw. **I:**

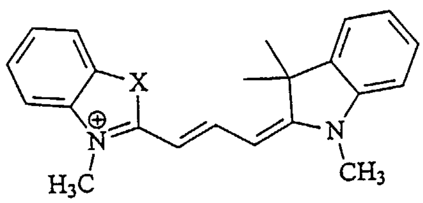

**H**

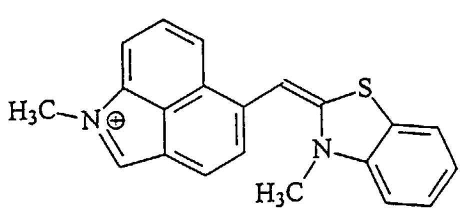

**I**

Der erste (**H**; X = S) hat ein Absorptionsmaximum bei 542 nm (in Ethanol), der zweite bei 545 nm, obwohl sich zwischen den chromophoren Endgruppen (also den beiden Stickstoffatomen) beim erfindungsgemäßen neuen Molekül **I** ein kürzeres System (nur 3 C-Atome) befindet.

Ähnliches gilt für die beiden folgenden Farbstoffe **J** und **K**,

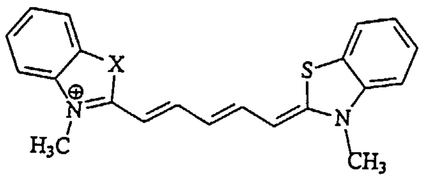

J

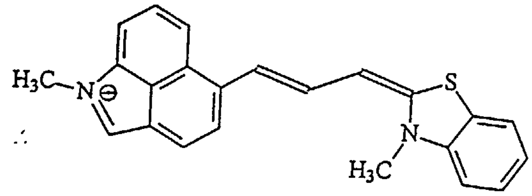

K

deren Absorptionsmaxima bei 650 bzw. 658 nm liegen, also trotz unterschiedlicher Moleküllänge nur unwesentlich verschieden sind.

Die Synthese der neuen Farbstoffe erfolgt vorzugsweise auf die im Folgenden skizzierte Weisen: Aus dem 5(1H)Benzo(c,d)indol-5-onen vom Typ **II** entstehen über die Thioverbindungen vom Typ **III** die kationischen Thioether vom Typ **IV:**

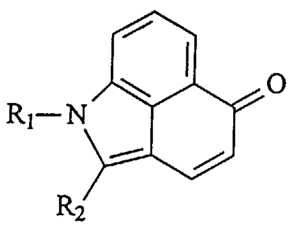

II

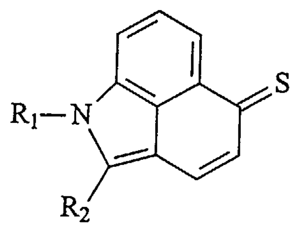

III

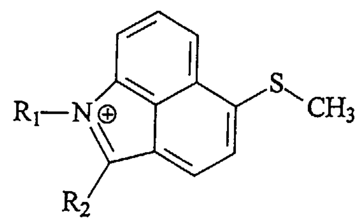

IV

Die Verbindungen **IV** können z. B. mit quarternierten Benzthiazolen der Struktur **V** (mit X = H oder Sulfo) umgesetzt werden und ergeben dann Farbstoffe vom Typ **VI:**

IV +

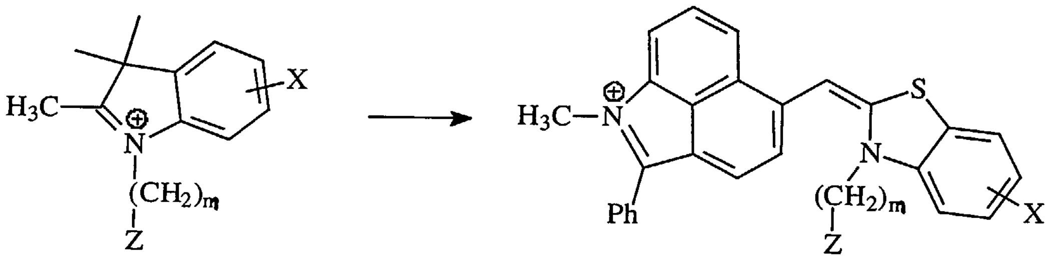

| | |
|---|---|
| Z = COOH | m = 5 |
| Z = CO-O-SUI | m = 5 |
| Z = OH | m = 6 |
| Z = PAM | m = 6 |

Um die obige Farbstoffklasse konjugierbar zu machen, ist es erforderlich, sie in eine reaktive Form zu überführen. Dies geschieht durch Einführung einer der in Tabelle 1 genannten reaktiven Gruppen. So kann man z. B. eine Sulfo-Gruppe (Verbindung VI; X = $SO_3^-$) in ihr Sulfochlorid überführen.

Wenn z.B. in Verbindung **VI** n = 5 und Z = COOH, hat man einen farbigen bzw. fluoreszenten Farbstoff in der

Hand, der leicht in einen reaktiven N-Hydroxysuccinimidester überführt werden kann:

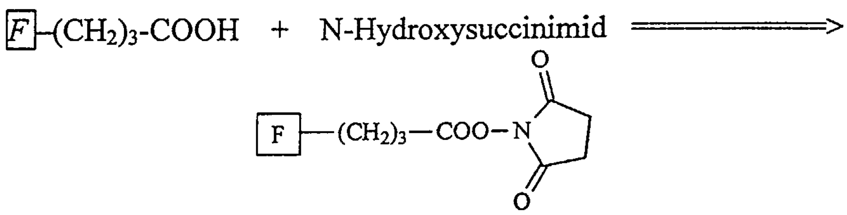

Dieser NHS-Ester reagiert z. B. mit Aminogruppen von Biomolekülen wie folgt:

$H_2N$-Biomolekül = = > *F* - NH-Biomolekül + NHS

Auf diese Weise erhält man mit *F* markierte Biomoleküle.

Wählt man hingegen als *Z* in Verbindung **VI** eine OH-Gruppe, so kann man sie über eine Phosphoramidit-Gruppe so aktivieren, dass sie mit Ribosen bzw. Desoxyribosen stabile Konjugate ergeben. Dazu wird der Alkohol-Fluorophor *F* -$(CH_2)_n$-OH zuerst mit einem Phosphoramidit **(I)** zu **II** umgesetzt, dieses dann mit einem geschützten Nucleosid in bekannter Weise zu **III.** Nach Oxidation mit Iod erhält man fluoreszenz-markierte Nucleoside vom Typ **IV.** Diese Reaktion ist im Folgenden dargestellt, wobei *B* für eine Nucleobase steht.

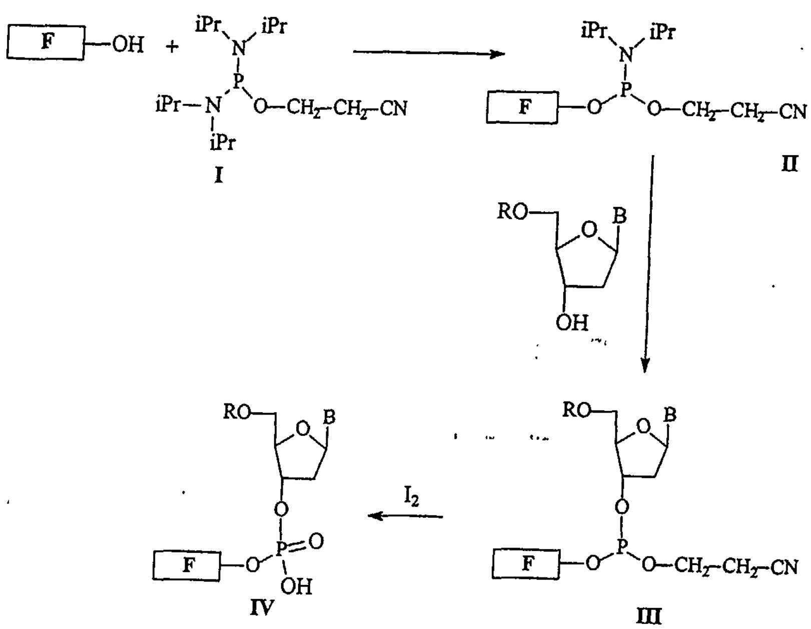


I

II

III

IV

**Beispiele**

Die folgenden Beispiele zur Herstellung erfindungsgemäßer Chromophore, deren Einsatz in der Markierung von Biomolekülen sowie deren Einsatz in fluoreszenz-analytischen Bestimmungsverfahren für Biomoleküle.

Beispiel 1 Herstellung von 1-Methyl-2-phenyl-5(1H)-benzo[c,d]indolon (Verbindung 259)

2,51 g Dimethylsulfat werden zu einer Lösung von 2,45 g (10 mMol) 5-Hydroxy-2-phenylbenzo[c,d]indol in 50 mL 10%-iger Natronlauge getropft. Man rührt 2 Stunden, worauf ein Niederschlag entstanden ist, der filtriert wird. Ausbeute 2,18 g (61%) an schwachgelben Kristallen mit einem Schmp. >250 °C.

Beispiel 2 Herstellung von 1-Methyl-2-phenyl-5(1H)-benzo[c,d]indol-thion (Verbindung 275)

Eine Mischung aus 2.6 g (10 mMol) 1-Methyl-2-phenyl-5(1H)-benzo[c,d]indolon (siehen oben), 10 mL Pyridin und 2,3 g $P_2S_5$ wird eine Stunde lang bei 100 °C heftig gerührt. Danach gießt man in 100 mL Wasser und filtriert den Niederschlag ab. Ausbeute: 2,1 g (76%) gelblicher Kristalle vom Schmp. > 250 °C.

Beispiel 3 Herstellung von 1-Methyl-5-methylthio-2-phenylbenzo[c,d]indolium-Iodid (Verbindung 417)

Eine Mischung aus 2,75 g (10 mMol) 1-Methyl-2-phenyl-5(1H)-benzo[c,d]indol-thion (Verbindung 275), 10 mL trockenem Aceton und 2,85 (20 mMol) Methyliodid wird 2 h unter Rückfluss erhitzt. Dann kühlt man ab und filtriert den entstandenen Niederschlag (1,75 g; 42%) ab. Gelbe Kristalle, Schmp. >250 °C.

Beispiel 4 Herstellung von 1-[5-(3-methyl-2(3H)benzothialzolyliden)-methyl-2-phenyl-benzo[c,d]indolium]-buttersäure-Iodid (Verbindung 532)

Eine Mischung aus 420 mg (1 mMol) von 1-Methyl-5-methylthio-2-phenyl-benzo[c,d]indolium-Iodid, 10 mL trockenem Ethanol, 290 mg (1 mMol) 4-(2-Methyl-1-benzthiazolium)buttersäure-Iodid und 100 mg (1 mMol) Triethylamin wird 10 min zum Sieden erhitzt. Danach kühlt man ab und filtriert den roten Niederschlag ab. Ausbeute: 410 mg (70%) roter Kristalle; Schmp. > 250 °C.

Beispiel 5 Herstellung eines reaktiven NHS-Esters (Verbindung 532-NHS)

In 1 mL trockenem und frisch destillierten Dimethylformid (DMF) wurde 14 mg Dicyclohexyl-carbodiimide (DCC) und 8 mg N-hydroxysuccinimide (NHS) gelöst. Dazu gab man 20 mg von Verbindung 532 (s. o.), ebenfalls in 1 mL DMF gelöst. Die Lösung wurde 8 h bei Raumtemperatur gerührt und danach filtriert. Das Lösungsmittel wurde unter Vakuum entfernt und der Rückstand auf einer RP-Chromatographiesäule aufgereinigt (Laufmittel Methanol). Die rot-violette Lösung wurde eingedampft und der Rückstand über $P_2O_5$ getrocknet. Ausbeute 20 mg (86%) einer rotvioletten Kristallmasse.

Beispiel 6 Herstellung eines Biotin-Konjugates (Verbindung 532-Biotin)

In 1 mL trockenem und frisch destillierten Dimethylformid (DMF) wurde 14 mg Dicyclohexyl-carbodiimide (DCC) und 8 mg N-hydroxysuccinimide (NHS) gelöst. Dazu gab man 20 mg von Verbindung **532** (s. o.), ebenfalls in 1 mL DMF gelöst. Diese Lösung wurde nun langsam in eine wässrige Lösung geleitet, die aus einer Lösung von jeweils 8.4 mg Biotinhydrazid (Sigma, Prod. Nr. B-7639) oder Biotin-Ethylendiamin (= N-(2-aminoethyl)biotinamide hydrobromide; Produkt Nr. A-1593 der Fa. Molecular Probes; Portland, Oregon) in 10 mL Phosphatpuffer von pH 8.0 bestand. Man lässt 2 h bei 25 °C stehen, fügt einige Tropfen einer 1 M Salzsäure zu und zieht das Lösungsmittel weitgehend ab. Das feste Produkt wird in Methanol gelöst und auf einer 20-cm-Sephadex-Säule (G50) gel-chromatographisch gereinigt.

Beispiel 7 Markierung von Humanserumalbumin mit den Reaktivfarbstoff 532-NHS

Man löste 1 mg des NHS-Esters **532-NHS** in 100 µL wasserfreiem DMF. Gleichzeitig wurden 5 mg Humanserumalbumin (HSA) in 1 mL einer 50 mM Bicarbonate-Pufferlösung von pH 9.0 gelöst. Die DMF-Stammlösung wurde nun in kleinen Portionen (30 µL) und unter Rühren in die Proteinlösung gegeben. Nach beendeter Zugabe wurde 5 h weitergerührt.

Unkonjugierter Farbstoff wurde vom markierten Protein durch Gelpermeations-Chromatographie mit 22 mM Phosphatpuffer von pH 7.2 getrennt. Die am schnellsten laufende rote Bande enthielt das markierte Protein.

Beispiel 8 Markierung eines amino-modifizierten Nucleinsäure-Oligomers

Das Amino-modifizierte 15-Oligomere 3'-TAA-TGG-CCT-GAG-ATAT-$(CH_2)_6$-$NH_2$ wurde mit dem Reaktivfarb-

stoff 532-NHS auf folgende Weise umgesetzt: Das Oligomer (0.2 mg) wurde in 5 mL Acetonitril gelöst und auf 50 °C erwärmt. Danach wurde eine Lösung von 0.1 mg des Farbstoffes in 1 mL Acetonitril langsam zugetropft. Nach 1 h wurde das Acetonitril abgedunstet und der Rückstand einer Polyacrylamid-Elektrophorese unterworfen. Oligomer und restlicher (überschüssiger) Farbstoff lassen sich leicht trennen.

Beispiel 9 Markierung von Glas-Mikropartikeln Poröse Glaskügelchen (1.0 g; Porengröße 70 nm; mit Aminopropylgruppen an der Oberfläche (40 - 100 µMol pro Gramm Kügelchen; bezogen von Sigma, Produkt-Nr. G-5019) wurde in Bicarbonat-Pufferlösung von pH 8.0 suspendiert. Dazu wurde langsam und unter raschem Rühren bei 50 °C eine Lösung von 3 mg des Farbstoffes **532-NHS** in DMF zugetropft. Nach einer Stunde wurden die rot angefärbten Glaspartikel abgetrennt, mit reichlichen Mengen an destilliertem Wasser, 1 %-iger Essigsäure und wieder Wasser gewaschen, bis kein Farbstoff mehr im Waschwasser zu finden war. Danach wurden die Partikel getrocknet und im trockenen Zustand gelagert. Die rot gefärbten Partikel weisen eine starke gelbgrüne Fluoreszenz auf.

Beispiel 10 Markierung eines Proteins mit einem Biotinfarbstoff

Die Markierung erfolgte über die Biotin-Streptavidin-Bindungsreaktion. Polyclonales anti-HSA (Ziege; Sigma Prod. Nr. A-1151) wurde nach 10-facher Verdünnung mit Phosphat-Puffer zuerst mit Streptavidin-Maleinimid (Sigma, Prod. Nr. S-9415) nach der Vorschrift von Duncan et al. in *Analytical Biochemistry* **132** (1983) 68 markiert. Das so entstandene Streptavidin/anti-HSA-Konjugat wurde nach elektrophoretischer Aufreinigung in Pufferlösung (pH 7.0) mit dem Biotin-Reagens **532-Biotin** (s. o.) versetzt und nach 2h Stehen bei Raumtemperatur elektrophoretisch gereinigt. Man erhält auf dieses Weise einen orangeroten Antikörper mit einer schwach gelbgrünen Fluoreszenz.

**Patentansprüche**

1. Farbige und fluoreszente Markerfarbstoffe mit einem Absorptionsmaximum zwischen 420 und 950 nm der allgemeinen Struktur 1

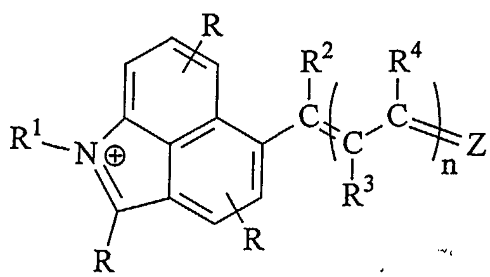

**1**

worin
R für H oder einen organischen Substituenten oder Ring steht,
$R^1$ bis $R^4$ für H, einen gegebenenfalls substituierten linearen, verzweigten oder cyclischen, gesättigten oder ungesättigten Alkylrest, oder einen gegebenenfalls substituierten Arylrest stehen kann,
$R^2$ bis $R^4$ auch für eine Halogen-, Cyano- oder eine gegebenenfalls substituierte Aminogruppe stehen kann,
R und $R^1$ bis $R^4$ über carbocyclische oder heterocyclische Ringe miteinander verbunden sein können;
Z für einen zweiwertigen aromatischen oder heteroaromatischen Rest steht, der auch einen Rest R, wie er oben definiert ist, enthalten kann,
*n* Werte zwischen 0 und 3 annehmen kann, und
zumindest ein Rest R, bzw. $R^1$ bis $R^4$ eine Gruppe enthält, die eine Markierung eines Biomoleküls oder eines Partikels ermöglicht.

2. Fluoreszente Markerfarbstoffe nach Anspruch 1,
**dadurch gekennzeichnet**,
1, dass mindestens einer der Substituenten R bzw. $R^1$ bis $R^4$ eine kovalente oder nichtkovalente Bindung des Markerfarbstoffes an ein Biomolekül eingehen kann.

3. Fluoreszente Markerfarbstoffe nach Anspruch 1 oder 2,

**dadurch gekennzeichnet,**
**dass** mindestens ein Substituent R entweder
ein reaktiver Ester einer Carbonsäure, ein Maleinimid, ein Haloacetyl vom Typ -CO-$CH_2$-X, worin X für Halogen steht, ein Sulfochlorid, ein Chlorotriazin, eine -SCN oder -OCN-Gruppe, eine Pyryliumgruppe, oder ein Biotin ist.

**4.** Fluoreszente Markerfarbstoffe nach Anspruch 1 oder 2,
**dadurch gekennzeichnet,**
**dass** an mindestens einem der Substituenten R oder $R^1$ bis $R^4$ ein reaktiver Ester einer Carbonsäure, ein Maleinimid, ein Haloacetyl vom Typ -CO-$CH_2$-X, worin X für Halogen steht, ein Sulfochlorid, ein Chlorotriazin, eine -SCN oder -OCN-Gruppe, eine Pyryliumgruppe, oder ein Biotin vorhanden ist.

**5.** Fluoreszente Markerfarbstoffe nach einem der Ansprüche 1 bis 4,
**dadurch gekennzeichnet,**
**dass** das Molekül eine ionische Gruppe, vorzugsweise eine Sulfo-, Sulfat-, Phosphat-, Phosphonat- oder quarternierte AmmoniumGruppe enthält.

**6.** Verwendung eines reaktiven Markerfarbstoffs nach einem der Ansprüche 1 bis 5 zur Herstellung von fluoreszent markierten Biomolekülen, insbesondere von Proteinen oder Antikörpern, von DNA, RNA oder PNA, von Pharmaka oder Zuckern, von Zellen oder Gewebsschnitten, bzw. von organischen Partikeln mit einem Durchmesser zwischen 0.01 µm und 10 µm.

**7.** Fluoreszente Biomoleküle, insbesonders Proteine oder Antikörper, DNA, RNA oder PNA, Pharmaka oder Zucker, bzw. fluoreszente organische Partikeln mit einem Durchmesser zwischen 0.01 µm und 10 µm, **dadurch gekennzeichnet, dass** sie mit einem fluoreszenten Marker nach einem der Ansprüche 1 bis 5 markiert sind.

**8.** Verwendung eines fluoreszenten Biomoleküls oder Partikels nach Anspruch 7 in fluoreszenz-analytischen Bestimmungsverfahren.

**9.** Verwendung nach Anspruch 8,
**dadurch gekennzeichnet,**
**dass** das Bestimmungsverfahren auf der Messung der Intensität, der Abklingzeit, der Effizienz des Energietransfers oder der Polarisation der Fluoreszenz beruht.

**10.** Verwendung nach Anspruch 8 oder 9,
**dadurch gekennzeichnet,**
**dass** das Bestimmungsverfahren unter Verwendung einer Mikrotiterplatte, eines Fließcytometers oder eines anderweilig automatisierten oder automatisierbaren Laborverfahrens durchgeführt wird.